# EUROPEAN PATENT APPLICATION

(11) **EP 4 759 283 A1**
(43) Date of publication of application: **17.06.2026**
(21) Application number: 24219496.7
(22) Date of filing: 12.12.2024
(51) Int. Cl.: A61F 13/49, A61F 13/505

(54) **WASHABLE ABSORBENT ASSEMBLY WITH BREATHABLE BARRIER MEMBER**

(71) Applicant: Essity Hygiene and Health Aktiebolag, 405 03 Göteborg (SE)
(72) Inventor: KALENTUN, Pia, 405 03 GÖTEBORG (SE); FERNKVIST, Maria, 405 03 GÖTEBORG (SE)
(74) Representative: Essity Hygiene and Health AB

(57) **Abstract**

Provided herein is a washable absorbent assembly (10) adapted to be permanently or detachably secured in a textile undergarment (1), the washable absorbent assembly (10) extending a length in a longitudinal direction and a width in a transverse direction, and having a thickness in a z-direction perpendicular to the longitudinal and transverse direction. The washable absorbent assembly (10) having a liquid permeable layer (101), a liquid barrier member (103) including at least one layer, and an absorbent member (102) arranged between the liquid permeable layer (101) and the liquid barrier member (103). Further, the liquid barrier member (103) has a breathability level in a range of of 2500 to 5500 g/m²/24 hours as measured according to Nonwoven Standard Procedure NWSP 70.4R0 (15). Also provided herein is a washable absorbent hygienic underwear for absorbing urine, menses, or other body fluids having a textile undergarment and in which a washable absorbent assembly (10) as described herein is permanently secured in the textile undergarment.

## Description

### Field of the invention

The present disclosure relates to washable absorbent assemblies that are adapted to be permanently or detachably secured in an undergarment and which are designed to accommodate a balance between fluid handling properties (e.g., management of absorbed fluids) and user comfort. In particular, these washable absorbent assemblies are configured to have a breathability level that enables the provision of an improved microclimate on an interior skin-facing side of the absorbent assembly and that also maintains a high level of security against leakage on an exterior garment-facing side of the absorbent assembly. Also provided herein are washable absorbent hygienic underwear with which the washable absorbent assemblies are integrated.

### Background

It is generally well known that disposable absorbent articles, such as sanitary napkins, panty liners, diapers, incontinence pads, and the like need to have good absorptive properties and should provide the wearer with a sense of security against leakage. Such disposable absorbent articles are generally constructed with a liquid permeable topsheet, a liquid impermeable backsheet, and an absorbent structure arranged between the topsheet and the backsheet. The absorbent structures are typically formed with an absorbent core that contains superabsorbent material, like superabsorbent polymers (SAP). These superabsorbent materials contribute to the fluid handling characteristics of the absorbent articles and may provide, for example, relatively high absorption capacity and relatively high liquid retention capacity.

To be appealing to consumers, it is also necessary for the absorbent articles to provide a relatively high level comfort during use thereof. In this regard, it is particularly important that the conditions of the microclimate, which is the condition of the air gap between the interior skin-facing side of the absorbent article and the wearer, are satisfactory to perceive comfort. Occlusion of the skin can lead to increased humidity which, in turn, can increase growth of microorganisms, enhance skin degradation, facilitate skin penetration of potentially toxic substances, and increase the risk for chaffing. Therefore, it is desirable that disposable absorbent articles are breathable.

To impart and/or enhance breathability, disposable absorbent products are often provided with backsheet material(s) that is/are liquid-impermeable yet breathable. A breathable backsheet permits diffusion of moisture vapor (i.e., moisture in the gaseous phase) therethrough but does not allow liquid to pass. This is beneficial because the presence of too much water vapor can make the microclimate between the absorbent article and the skin of the wearer too humid. When the microclimate is too humid, this can cause irritation of the skin in contact with the microclimate. By allowing water vapor to diffuse out from the backsheet, the humidity of the microclimate can be regulated and thus minimize, if not avoid, the risk of skin irritation.

A vast majority of the disposable absorbent articles that are currently commercially available contain a significant amount of material(s) sourced from petrochemicals, such as petroleum-based fibers and petroleum-based polymers, usually in both the topsheet and backsheet layers. Not to mention, the superabsorbent materials used to form the absorbent structure and/or additional layers are also often derived from petroleum-based polymers or materials. These materials are not sustainable and contribute to an ever-growing waste stream of used absorbent articles that ultimately end up in traditional landfills. Thus, there is an existing demand to shift toward alternative sustainable solutions to remediate the impact of such disposable absorbent articles on available resources and the environment.

Reusable absorbent undergarments, which may be worn for the purpose of absorbing bodily fluids such as urine and vaginal fluids, have recently increased in popularity to supplement and/or as an alternative to single-use disposable absorbent articles. Such garments offer advantages in terms of alleviating the amount of resources expended in manufacturing traditional disposable absorbent articles as well as reducing the waste stream that follows from use of such articles. Further still, reusable absorbent undergarments may also be considered advantageous from an economic standpoint since the reusability of these products lessens the amount of article(s) consumers need to buy.

Conventional reusable absorbent undergarments typically have an integral absorbent assembly arranged in a crotch region of the undergarment. After use, the absorbent undergarment may be laundered, and thereafter, can be reused again. WO 2023/169665 A1 describes one such exemplary absorbent undergarment.

Unlike the absorbent structures of their disposable counterparts, the absorbent assemblies of washable absorbent undergarments are not constructed with superabsorbent materials since such material(s) is/are essentially incompatible with laundering (i.e., washing and drying) of the undergarment. In other words, it would be rather difficult, if not near impossible, to wash and dry a used absorbent assembly that contains superabsorbent material(s) for repeat use since superabsorbent material(s) are structured to absorb and, more importantly, to retain relatively large amounts of fluids (including liquid(s) involved in the laundering process), not to expel them.

In the absence of superabsorbent material(s), and in order to secure against leakage from the reusable absorbent undergarment, the washable absorbent assemblies may be provided with a watertight barrier layer that is arranged beneath the absorbent layer(s) of the absorbent assembly; this is to prevent or at least hinder passage of absorbed fluid(s) from the absorbent assembly and out of the absorbent undergarment and into contact with additional clothing layer(s) and/or the skin of the wearer. However, employing such watertight barrier layers is not without trade off, particularly in that these watertight barrier layers are not sufficiently breathable. As a result, the microclimate between the wearer and currently available absorbent undergarments is not well-regulated, which increases the risk for skin irritation; not to mention, there is an increased likelihood that the level of comfort will decrease as fluid(s) is/are absorbed and the absorbent undergarment will become uncomfortable to the wearer.

Therefore, there exists a need to provide washable absorbent assemblies that not only possess efficient fluid handling properties that are at least comparable to those of conventional disposable absorbent articles, but that also do so while improving user comfort. In this sense, it is highly desirable to provide washable absorbent assemblies that are sufficiently breathable to optimize the microclimate between the absorbent assembly and the wearer during use and/or minimize, if not eliminate, the risk of skin irritation.

### Summary

Despite existing solutions for washable absorbent articles available to supplement and/or use in place of single-use disposable absorbent articles, it has been found that there is still a need to improve the level of user comfort associated with these articles during use. In this regard, it is important that the washable absorbent assemblies are adapted to facilitate regulation of the microclimate between skin of a wearer and the absorbent assembly. It is, therefore, highly desirable that the washable absorbent assemblies are adequately breathable to enhance the microclimate and/or minimize, if not all together eliminate, the risk of skin irritation.

The breathability of the washable absorbent assemblies should be adapted such that the microclimate between an interior skin-facing side of the absorbent assembly and the skin of the wearer is minimally, if not, not at all, damp and/or cold. It further follows that the washable absorbent assemblies should also not give a damp and/or cold feeling exterior to the absorbent assembly (i.e., between a garment-facing side of the absorbent assembly and any additional layer(s) of clothing and/or the wearer).

Further, it is desirable that the washable absorbent assemblies are enabled to effectively capture and retain absorbed fluid(s) in a desired and predetermined manner, and by extension, prevent leakage therefrom, all while maintaining a fresh feeling for the wearer. In this sense, the washable absorbent assemblies should also provide the wearer with a continued sense of dryness during use of the absorbent assembly.

An objective of the present disclosure is thus to provide a washable absorbent assembly having improved wearer comfort, both on an interior skin-facing side and on an exterior garment-facing side of the absorbent assembly. In this regard, the absorbent assembly should be enabled to provide an enhanced microclimate between the absorbent assembly and skin of the wearer during use by regulating, for example, the temperature and/or relatively humidity on an interior skin-facing side of the absorbent assembly. In addition, the absorbent assembly should be adapted such that, during use, the wearer is not given a damp and/or cold sensation on the exterior garment-facing side of the absorbent assembly, for example, by controlling the amount of accumulated moisture vapor permitted to diffuse out of the absorbent assembly without impact to the imperviousness of the absorbent assembly to absorbed fluid(s) leakage.

In terms of breathability, it is desirable that the absorbent assembly is adapted such that its breathability is relatively consistent regardless of the state (i.e., wet or dry) of the absorbent assembly. In other words, there should be minimal variation in the breathability characteristics of the absorbent assembly, and overall absorbent undergarment into which the absorbent assembly is integrated, during use, whether the absorbent assembly is dry, wet, or somewhere in between. This may be referred to herein as a balanced breathability or a medium breathability, and it is to be understood that these terms may be used interchangeably.

It is also an objective to provide a washable absorbent assembly in which leakage security is not reduced, if not all together unaffected.

In terms of fluid handling, it is desirable that the absorbent assembly is enabled to minimize, if not eliminate, the occurrence of surface wetness at any point during use. Further, it is desirable that the absorbent assembly has a relatively high capacity for absorption and that body liquid does not leak out and risks wetting the user's clothing.

It is further desirable still to provide an absorbent assembly that is discrete and/or aesthetically pleasing to wear. Any one or more of the aforementioned properties may additionally contribute to and/or improve the comfort level of a user when wearing the absorbent assembly.

Depending on the desired application, the washable absorbent assemblies described herein may take any form that is suitable for either permanent or detachable securement in an absorbent undergarment. Further, it is to be understood that the absorbent undergarment itself may also take any suitable form, such as those including but not limited to, underpants, briefs, panties, and the like.

The objectives set forth herein are achieved by the present washable absorbent assembly as defined in the appended claims.

Accordingly, in a first aspect of the present disclosure there is provided a washable absorbent assembly adapted to be permanently or detachably secured in a textile undergarment. The washable absorbent assembly extends a length in a longitudinal direction (y) and a width in a transverse direction (x) and has a thickness in a z-direction perpendicular to the longitudinal and transverse directions (x, y). The washable absorbent assembly comprises a liquid permeable layer, a liquid barrier member comprising at least one layer, and an absorbent member arranged between the liquid permeable layer and the liquid barrier member. Further, the liquid barrier member has a breathability level in a range of 2500 to 5500 g/m² /24 hours as measured using a PERMATRAN-W Model 101K (available from Mocon, Inc., Minneapolis, MN) or equivalent, according to Nonwovens Standard Procedure NWSP 70.4. R0 (15) with the following specifications: experiments were carried out in a lab controlled at 23 °C ± 2 °C and 50 %RH ± 2 %RH and the instrument cells heated to 37.8°C (100°F).

Adapting the absorbent assembly to have a liquid barrier member that has a breathability level in a range of 2500 to 5500 g/m² /24 hours contributes to improving the level of comfort afforded to a wearer during use of the undergarment to which the absorbent assembly is secured. Specifically, a breathability level in a range of 2500 to 5500 g/m² /24 hours has been found to permit the diffusion of accumulated water vapor through an exterior garment-facing side of the absorbent assembly without impact to the imperviousness of the liquid barrier member. Advantageously, this further contributes to enhancing the microclimate between the skin-facing side of the absorbent assembly and skin of the wearer because the release of moisture vapor from the absorbent assembly assists to regulate the temperature and/or relative humidity on the skin-facing side of the absorbent assembly. Thus, it is possible to minimize, if not avoid, the microclimate becoming too humid during use of the absorbent assembly.

Also, a further benefit of regulating the microclimate between the absorbent assembly and skin of the wearer is that it is possible to minimize, if not avoid, the risk of skin irritation.

In addition, providing a liquid barrier member with a breathability level in a range of 2500 to 5500 g/m² /24 hours further contributes to providing the wearer with a continued sense of freshness of the article, thereby improving the overall perceived comfort. More specifically, this level of breathability assists to ensure that only moisture vapor is diffused through the liquid barrier member and not absorbed fluid(s). This advantageously minimizes, if not all together eliminates, the risk that the wearer will experience a damp and/or cold sensation exterior to the absorbent undergarment. As such, the wearer can expect to instead experience a fresh and dry sensation while wearing the absorbent undergarment.

In certain non-limiting exemplary embodiments, it may be preferable that the liquid barrier has a breathability level in a range of 3000 to 5000 g/m² /24 hours. Again, such level of breathability may further contribute to and/or enhance user comfort of the absorbent undergarment to which the absorbent assembly is secured. Likewise, breathability within the preferred range is also expected to facilitate diffusion of accumulated moisture vapor through the garment-facing side of the liquid barrier member without sacrifice to the security against leakage provided by the absorbent assembly, and more specifically, by its liquid barrier member.
The liquid barrier member functions to prevent absorbed fluid(s) and/or moisture in the liquid state from penetrating through a garment-facing side of the absorbent assembly and into contact with additional layer(s) of clothing and/or the wearer. It is also important that the liquid barrier member is *washable* as defined as having an ability of withstand laundering at 40 °C at least 100 times without any appreciable loss in appearance, integrity and/or functionality. When used herein laundering means that the absorbent assembly may be subjected to an aqueous solution containing detergent.

In non-limiting exemplary embodiments, the liquid barrier member may comprise a plastic film. Suitable materials may include thermoplastic films formed from polymers, including but not limited to, polyolefin-based polymers, such as polyurethane, polypropylene, or the like. However, it is to be understood that the specified materials are exemplary and that one of ordinary skill in the art may readily select one or more other suitable materials not specifically mentioned herein, provided that such material(s) exhibit a breathability level within the requisite ranges described herein and remain impervious to liquid leakage therefrom.

In some non-limiting exemplary embodiments, the liquid barrier member may be a microporous plastic film. It may be preferred that the microporous film is a polyethylene, a polytetrafluoroethylene (PTFE) or polyurethane film, or any combination(s) thereof. Again, it is to be understood that it is within the purview of the skilled person to select any suitable material comparable to those specified mentioned herein.

Further, as measured using a PERMATRAN-W Model 101K (available from Mocon, Inc., Minneapolis, MN) or equivalent, according to Nonwovens Standard Procedure NWSP 70.4. R0 (15) with the following specifications: experiments were carried out in a lab controlled at 23 °C ± 2 °C and 50 %RH ± 2 %RH and the instrument cells heated to 37.8°C (100°F).

In non-limiting examples, the absorbent member may comprise two or more structures. Particularly, the absorbent member may comprise a liquid retaining structure and a liquid acquisition structure which is arranged between the liquid permeable layer and the liquid retaining structure.

It is further contemplated that the liquid barrier member may, in non-limiting exemplary embodiments take the form of a laminate. It may generally be preferred, in certain embodiments, that the laminate comprises a plastic film and at least one additional layer. For example, laminates of polymer films, including those specifically mentioned herein, and non-woven material(s) may be used.

When the liquid barrier member is provided in the form of a laminate, particularly of the type that comprises a plastic film and at least one additional layer, it may be preferable that the at least one additional layer is a textile layer. Such textile layer(s) may comprise any material(s) suitable for absorbing fluid(s) and/or exudates, such as woven or nonwoven material(s) like microfiber or polymer knits fabric formed from hydrophilic fibers, other absorbent fibers, combinations, and/or composites thereof. Additionally, the material(s) may be natural, synthetic, or combinations thereof.
Structurally, when the liquid barrier member is provided in the form of a laminate, also of the type that comprises a plastic film and at least one additional layer which may be a textile layer, it may be further preferable that the textile layer is arranged proximal to the absorbent member. Stated in different terms, in certain non-limiting exemplary embodiments, it is preferable that the plastic film is arranged proximal to the garment-facing side. Arranging the laminate in this manner is advantageous because this contributes to the overall security against leakage of the absorbent assembly since the bulk of the absorbed fluids are retained in a portion of the assembly that is not directly adjacent to the garment. There is some intervening layer between where the absorbed fluid(s) are primarily stored and the outermost layer of the garment-facing side of the absorbent assembly. An additional textile layer arranged proximal to the absorbent member may be hydrophobic or hydrophilic. A benefit with a hydrophobic textile layer proximal to the absorbent member is that a hydrophobic textile layer creates a distance between the absorbent member and the outermost layer of the garment facing side of the absorbent assembly.
According to an alternative embodiment is the textile layer arranged proximal to the garment facing side. According to this embodiment is the textile layer preferably hydrophobic.
According to a further alternative embodiment is the liquid barrier member provided in the form of a laminate comprising at least three layers. According to this embodiment is one textile layer arranged proximal to the absorbent member and one textile layer arranged proximal to the garment facing side. The textile layer arranged proximal to the absorbent member may be hydrophobic or hydrophilic, and the textile layer arranged proximal to the garment facing side is preferably hydrophobic.

The liquid acquisition structure may facilitate liquid handling into and within the absorbent member. In particular, the liquid acquisition structure facilitates initial intake and distribution of absorbed fluid(s) by providing a space in which discharged fluid(s) can be rapidly captured and temporarily stored. Discharged fluid(s) readily pass through the liquid permeable layer, are wicked away from its top surface that face the wearer, and are absorbed into the liquid acquisition structure 1021. This means that the absorbed fluids are not in contact with the wearer's skin and thus may provide the wearer with a feeling of dryness.

The liquid acquisition structure is also designed to moderate the flow of absorbed fluid(s) therethrough and into the absorbent layer(s) (e.g., liquid retention structure) arranged thereunder. By controlling the flow of fluid through the liquid acquisition structure, the liquid retention structure may be given time to process and efficiently distribute the absorbed fluid(s) therethrough. This provides efficient fluid management during use of the absorbent undergarment.

In addition, it is contemplated that the liquid retaining structure may, in certain non-limiting embodiments, generally have a higher density than the liquid acquisition structure.

Generally speaking, the density of an absorbent member is often proportional to its absorption capacity. Thus, a relatively high density typically correlates with a relatively high absorption capacity. Conversely, a relatively low density typically correlates with a relatively low or lower absorption capacity.

The liquid retaining structure and the liquid acquisition structure each have a respective density, which in preferred exemplary embodiments of the absorbent assemblies described herein are not the same or equal in magnitude. In certain non-limiting embodiments, the respective density of the liquid retaining structure may be at least 20% higher than the respective density of the liquid acquisition structure. In certain non-limiting embodiments, it may be preferable that the respective density of the liquid retaining structure is at least 50% higher than the liquid acquisition structure.

In non-limiting exemplary embodiments, the liquid acquisition structure may have a liquid retaining capacity of 0.2 g/cm² or less. On the other hand, the liquid retaining structure may have a liquid retaining capacity of 0.3 g/cm² or more.

It is contemplated that, in certain non-limiting examples, the washable absorbent assembly may be a washable absorbent pad adapted to be detachably secured to a textile undergarment. Further, it is to be understood that the textile undergarment can take the form of any suitable undergarment, including but not limited to any one of a textile undergarment.

Furthermore, it may be preferred, in certain exemplary non-limiting embodiments, that the washable absorbent assembly does not comprise superabsorbent material(s). Not including such superabsorbent material(s) in the construction of the washable absorbent assembly may avoid difficulty with or the diminishment of ease in laundering the absorbent assembly for repeat use.

In a further aspect of the present disclosure, there is provided a washable absorbent hygienic underwear for absorbing urine, menses, or other body fluids. The washable absorbent hygienic underwear has a length in a longitudinal direction (y), a width in a transverse direction (x), and a thickness in a z-direction perpendicular to the longitudinal and transverse directions (x, y) Here, the washable absorbent hygienic underwear may comprise any of the washable absorbent assemblies as described herein and a textile bottom comprising a front region, a back region, and an intermediate region arranged between the front and back regions, and further in which the washable absorbent assembly is permanently secured in the textile bottom.

Further objectives, features and advantages of the present absorbent article are described in the detailed description below with reference to the appended drawings.

### Brief description of the drawings

The present invention will be discussed in more detail below, with reference to the attached drawings, in which:
Figure 1 shows a front view of an exemplary absorbent undergarment 1 with an absorbent assembly 10 according to the present disclosure;
Figure 2 shows an exemplary an absorbent assembly 10; and
Figure 3 shows a cross-section of the absorbent assembly 10 of Fig. 2 taken along the line III.

### Detailed description

Different aspects of the present disclosure will be described more fully hereinafter with reference to the accompanying figures. The absorbent undergarment 1 provided herein can, however, be realized in many different forms, such as different sizes and/or in different absorption levels. Accordingly, the absorbent undergarment 1 should not be construed as being limited to the aspects set forth herein.

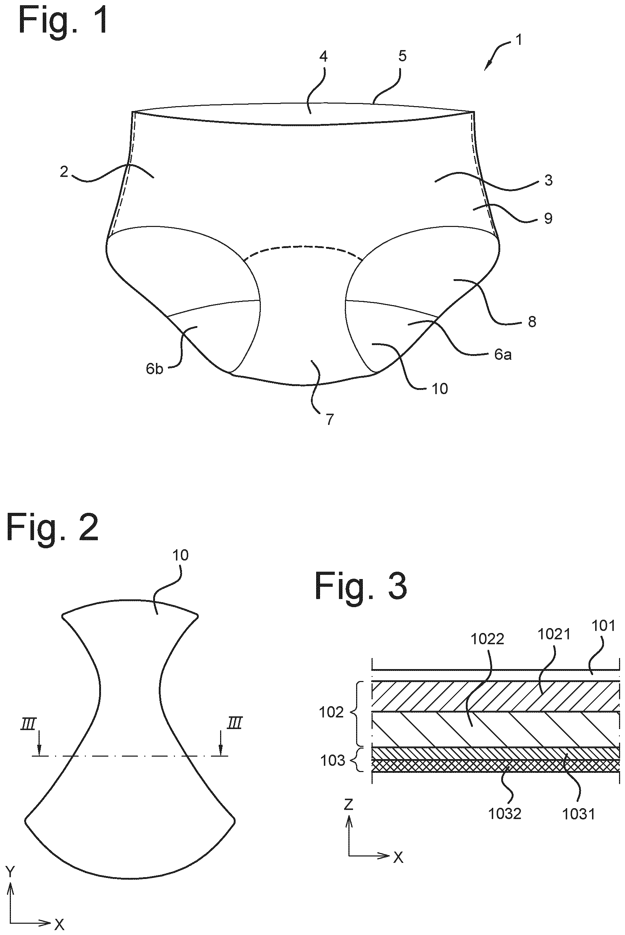

Figure 1 illustrates an exemplary washable and reusable absorbent undergarment 1. As indicated hereinabove, the absorbent undergarment 1 may take the form of various designs, including but not limited to, any of briefs, boxer, hipster, high waist, string, Brazilian, or other suitable undergarment designs. The undergarment may be designed for male, female, or even unisex use.

The absorbent undergarment 1 may comprise one or more fabric panels 2 forming a front region 3, a back region 4, and an intermediate region 7 extending between the front and back regions 3,4. The front region 3 and the back region 4 are joined such that the undergarment 1 forms a waist opening 5 and a pair of leg openings 6a, 6b. As such, the front region 3 will be visible from the front of the wearer when the undergarment is worn; likewise, the back region 4 will be visible from the back of the wearer.

The fabric panel 2 may be a single panel, which is cut to form the front region 3, the back region 4, and the intermediate region 7. However, in other variants, it is contemplated that a plurality of fabric panels 2 may be joined so as to form any one or more of the front region 3, the back region 4, and the intermediate region 7. For example, the undergarment may comprise a front panel, a back panel, and an intermediate panel. Further, the one or more fabric panels may all comprise the same fabric, giving the undergarment a unitary appearance. Alternatively, at least one of the individual fabric panels 2 may comprise a different fabrics than one or more of the other fabric panel. This may be done to provide aesthetically pleasing undergarments, for example, that comprise both functional fabric(s) and aesthetic fabrics (e.g., lace fabric).

The fabric of the one or more fabric panels 2 may be any suitable fabric, including naturally derived fibres and mixtures thereof selected from the group consisting of cotton, wool, silk, cellulose, regenerated cellulose, rayon, viscose, modal, lyocell, Tencel, bamboo, hemp, flax, ramie, coir or banana. Alternatively, the fabric may be constructed of synthetic fibres or mixtures thereof selected from the group consisting of polyamide, acrylic, polyester. Further, the fabric may be constructed of a blend or a mixture of naturally derived and/or synthetic fibres. The fibres may be recycled fibres. The fabric may comprise a stretchable fabric, such as elastane, so that the absorbent undergarment can provide a firm fit while simultaneously adapting to the movement(s) of the wearer, thus preventing any leakage from migrating through the leg openings and keeping the absorbent assembly in place. The fabric may also be breathable to allow vapor to escape from the skin of the wearer and from the absorbent structure.

As illustrated in Fig. 1, the fabric panel 2 has an outside surface 9 facing away from the wearer and an inside surface 8 facing in a direction towards the wearer. Further, an absorbent assembly 10 is arranged on the inside surface 8 of the intermediate region 7. A non-limiting example of the absorbent assembly 10 according to the present disclosure is depicted in Figure 2.

A cross-section of the exemplary absorbent assembly 10 taken along the line III of Fig. 2 is shown Figure 3. The absorbent assembly 10 generally comprises three components: a liquid permeable layer 101, an absorbent member 102, and a liquid barrier member 103. Structurally, the absorbent member 102 is arranged between liquid permeable layer 101 and the liquid barrier member 103.

The liquid permeable layer 101 lies in direct contact with the wearer's body and should therefore be soft and comfortable. The liquid permeable layer 101 may be constructed of any suitable fabric, including naturally derived fibers selected from any one or more of cotton, wool, silk, cellulose, regenerated cellulose, rayon, viscose, modal, lyocell, tencel, bamboo, hemp, flax, ramie, coir or banana. Alternatively, the liquid permeable layer 101 may be constructed of synthetic fibers including any one or more of polyamide, acrylic, polyester or elastane, such as a mixture of polyester and elastane. It is further conceivable that the liquid permeable layer 101 may also be constructed of a blend or a mixture of naturally derived and/or synthetic fibers. In any case, the material(s) used for construction of the liquid permeable layer 101 should be soft and non-irritating to the skin and should be readily penetrable by any body fluid(s), e.g., urine or menstrual fluid. Furthermore, the liquid permeable layer 101 should also be formed of material(s) that is capable of being repeatedly washed.

The liquid barrier member 103 is arranged on the garment-facing side of the absorbent assembly 10 and thus lies in contact with the wearer's other garments during use. As discussed herein, the liquid barrier member 103 is a liquid impervious structure designed to prevent absorbed fluid(s) and/or moisture in the liquid state from passing through the absorbent assembly 10 to any additional layer(s) of the absorbent undergarment 1, in which the absorbent assembly 10 is secured, thereunder and possibly further through to any additional clothing layer(s) and/or into contact with the wearer.

In addition to being liquid impervious, the liquid barrier member 103 is also breathable. Specifically, the liquid barrier member 103 is adapted to have a breathability level in a range of 2500 to 5500 g/m²/24 hours; it is contemplated that the liquid barrier member 103 may have a breathability level in a range of from 3000 to 5000 g/m² /24 hours in some non-limiting but preferred exemplary embodiments. Regardless, the level(s) of breathability of the liquid barrier member 103 described herein contribute(s) to improving the level of comfort afforded to a wearer. Specifically, a liquid barrier member 103 having a breathability level in a range of 2500 to 5500 g/m² /24 hours, or further preferred range thereof, allows diffusion of accumulated water vapor through the liquid barrier member 103 on a garment-facing side of the absorbent assembly 10 without impact to the imperviousness of the liquid barrier member 103 to absorbed fluid(s). At the same time as being breathable, the liquid barrier members 103 as described herein also prohibit the passage of absorbed fluid(s) and/or moisture in the liquid state from escaping through the liquid barrier member 103.

Consequently, liquid barrier members 103 having these specific breathability characteristics advantageously enhance the microclimate between the skin-facing side of the absorbent assembly 10 and skin of the wearer. In effect, the release of moisture vapor from the absorbent assembly 10 assists to regulate the temperature and/or relative humidity on the skin-facing side of the absorbent assembly 10. Thus, it is possible to minimize, if not avoid, the microclimate becoming too humid during use of the absorbent assembly 10. Regulation of the microclimate may, by extension, be understood to further minimize, if not eliminate, the risk that the wearer's skin may become irritated during or as a result of use of the absorbent undergarment.

Further, liquid barrier members 103 having the breathability characteristics as described herein also provide the wearer with a continued sense of freshness of the absorbent undergarment. More specifically, breathability within range of the requisite range(s) set forth herein assist to ensure that the liquid imperviousness of the liquid barrier member 103 is maintained such that only moisture vapor is diffused through the liquid barrier member 103 and not absorbed fluid(s). This minimizes risk that the wearer will experience a damp and/or cold sensation exterior to the absorbent undergarment during use thereof, and may further contribute to improving the perception of comfort of the absorbent undergarments and/or increase the wearer's confidence in the same.

It is contemplated that the liquid barrier member 103 can be constructed in a variety of non-limiting forms and with a variety of non-limiting source material(s), provided that the aforementioned liquid imperviousness and requisite breathability level(s) are satisfied.

For example, the liquid barrier member 103 can be suitably constructed of thermoplastic film(s) formed from polymers, including but not limited to, polyolefin-based polymers, such as polyurethane, polypropylene, or the like. Additionally or alternatively, the liquid barrier member 103 may comprise a plastic film 1032 which may or may be microporous. The plastic film 1032 may suitably have a basis weight in a range of from 10 to 50 gsm, and preferably from 20 to 40 gsm.

In certain non-limiting exemplary embodiments, such as the absorbent assembly 10 depicted in Figs. 2 and 3, the liquid barrier member 103 may take the form of a multi-layer structure and/or laminate. In Fig. 3, the exemplary liquid barrier member 103 is shown as a two-layer laminate structure. This laminate structure is formed by a plastic film 1032 and an additional layer 1031. As can be seen in Fig. 3, the additional layer 1031 is arranged between the plastic film 1032 and the absorbent member.

It may be preferable, in certain non-limiting exemplary embodiments, that the at least one additional layer 1031 is a textile layer. Such textile layer(s) may comprise any material(s) suitable for absorbing fluid(s) and/or exudates, such as woven or nonwoven material(s) like microfiber or polymer knits fabric formed from hydrophilic fibers, other absorbent fibers, combinations, and/or composites thereof. Additionally, the material(s) may be natural, synthetic, or combinations thereof.

Also, it may be further preferable in such non-limiting examples, that the at least one additional layer 1031, which is preferably a textile layer, is arranged proximal and/or directly adjacent to the absorbent member 102. Structurally, this arrangement may contribute to the overall security against leakage of the absorbent assembly 10 since a majority of the absorbed fluids may be retained in a portion of the absorbent assembly 10 that is not immediately adjacent to and/or or in direct contact with either fabric of the absorbent undergarment or another garment layer and/or skin of the wearer. A liquid barrier member 103 taking the form of a multi-layer structure and/or laminate may suitably have a basis weight in a range of from 20 to 80 gsm, or 20 to 50 gsm.

The absorbent member 102 is the absorbent structure of the absorbent assembly 10 which acquires and stores the absorbed bodily fluid(s) and exudate(s). The absorbent member 102 is also capable of releasing the absorbed fluid(s) and exudate(s) during laundering of the absorbent assembly 10. Release of absorbed fluid(s) is beneficial as it enable the absorbent assembly 10 to be restored for repeated use. Reusable absorbent assemblies and the undergarments in which such absorbent assemblies may be integrated provide a more sustainable alternative to commonly used single-use disposable absorbent hygiene articles.

The absorbent member 102 may comprise any material capable of absorbing fluid(s), such as woven or nonwoven material(s) like microfiber or polymer knits fabric formed from hydrophilic fibers, other absorbent fibers, combinations, and/or composites thereof. Additionally, the material(s) may be natural, synthetic, or combinations thereof. Also, it may be preferred in some non-limiting embodiments, that the absorbent member does not comprise superabsorbent material(s).

The absorbent member 102 may have a unitary construction. The phrase "unitary construction" in the present context is intended to mean that the absorbent member is constructed from essentially one type of material, this being essentially the same material, or essentially the same combination of two or more materials throughout the absorbent member. Variations in density and concentration of the material may occur, but these are limited to those which may be obtained without incorporation of regions which have been formed separately and then physically joined to each other. It is to be understood, however, that an absorbent member 102 of unitary construction does not comprise layers or laminates of different composition. Likewise, variations in the density or concentration of various components across the longitudinal direction, the transverse direction or the thickness direction of the absorbent member 102 are acceptable, yet the absorbent member 102 should not comprise areas or layers of different composition which are formed separately and later joined together.

Alternatively, it is also envisioned that the absorbent member 102 may be formed as a muti-layered structure and/or laminate. In this sense, the absorbent member 102 can comprise a plurality of absorbent layers and/or absorbent structures. When the absorbent member 102 comprises a plurality of absorbent layers and/or absorbent structures, the absorbent layers and/or absorbent structures may be equally sized or may differ in one or more dimensions.

Turning back to Fig. 3, the exemplary absorbent member 102 is shown as a two-layer laminate structure. This laminate structure is formed by a liquid acquisition structure 1021 and a liquid retaining structure 1022.

As can be seen in Fig. 3, the liquid acquisition structure 1021 is arranged between the liquid permeable layer 101 and the liquid retaining structure 1022. The liquid acquisition structure 1021 is adapted to quickly receive and temporarily store discharged fluid(s) before being absorbed into the liquid retaining structure 1022. Such liquid acquisition structure 1021 may be composed of, for example, any suitable fabric, including naturally derived fibers or mixtures thereof selected from any one or more of cotton, wool, silk, cellulose, regenerated cellulose, rayon, viscose, modal, lyocell, tencel, bamboo, hemp, flax, ramie, coir or banana. The liquid acquisition structure 1021 may also, in certain non-limiting exemplary embodiments, be constructed of synthetic fibers or mixtures thereof selected from any one or more of polyamide, acrylic, polyester or elastane. It is further envisioned that the liquid acquisition structure 1021 may, in certain non-limiting exemplary embodiments, be constructed of a blend or a mixture of naturally derived and/or synthetic fibers. In any case, the liquid acquisition structure 1021 should be launderable.

It is to be understood that the liquid acquisition structure 1021 can be formed from any one or more of the aforementioned materials.

It is also contemplated for any embodiment of the absorbent assemblies described herein that the liquid acquisition structure 1021 may be suitably dimensioned in the transverse and longitudinal directions (x, y) as desired, by one of ordinary skill, relative to the dimension(s) of the liquid retaining structure 1022. In this sense, the liquid acquisition structure 1021 may, in certain non-limiting examples, have a length in the longitudinal direction (y) and a width in the transverse direction (x) that are equal to or shorter than a respective length and respective width of the liquid retaining structure 1022. In other embodiments according to the present disclosure, the liquid acquisition structure 1021 and the liquid retaining structure 1022 may be co-extensive in the transverse and longitudinal directions (x, y).

On the other hand, the liquid retaining structure 1022 may be considered to form the primary absorbent portion of the absorbent assembly 10 and is adapted to securely retain absorbed fluid(s) during use of the absorbent assembly 10. Such liquid retaining structure 1022 may be constructed of, for example, any material capable of absorbing fluid(s), such as woven or nonwoven material(s) like microfiber or polymer knits fabric formed from hydrophilic fibers, other absorbent fibers, combinations, and/or composites thereof. Additionally, the material(s) may be natural, synthetic, or combinations thereof. In any case, the liquid retaining structure 1022 should also be configured to release the absorbed fluid(s) and exudate(s) during laundering so that the absorbent assembly 10 can be reused again and again.

It is to be understood that the liquid retaining structure 1022 can be formed from any one or more of the aforementioned materials, however, material(s) having a relatively high density, at least in comparison to the material(s) used to form the liquid acquisition structure 1021, may be particularly preferred in certain embodiments.

For example, a respective density of the liquid retaining structure 1022 may be at least 20% higher than the respective density of the liquid acquisition structure 1021. Additionally or alternatively, it may be further preferable still that the respective density of the liquid retaining structure 1022 is at least 50% higher than the liquid acquisition structure 1021.

Furthermore, the liquid acquisition structure 1021 and/or the liquid retaining structure 1022 may, in certain non-limiting embodiments, be additionally or alternatively characterized in terms of a respective liquid retaining capacity. For example, in certain non-limiting examples of the absorbent assemblies according to the present disclosure, the liquid acquisition structure 1021 may, in certain non-limiting examples of the absorbent assemblies according to the present disclosure, have a liquid retaining capacity of 0.2 g/cm² or less. In similar fashion, the liquid retaining structure 1022 may have a liquid retaining capacity of 0.3 g/cm² or more.

The washable absorbent assemblies described herein may take any form that is suitable for either permanent or detachable securement in an absorbent undergarment. In one non-limiting exemplary embodiment, the washable absorbent assembly 10 may take the form of a washable absorbent pad that is detachably secured to an absorbent undergarment, such as a textile pant. It is also to be understood that the absorbent undergarment itself may also take any suitable form, such as those including but not limited to, underpants, briefs, panties, and the like.

In a further aspect of the present disclosure, a washable absorbent assembly 10 as described herein is comprised in a washable absorbent hygienic underwear adapted for absorbing urine, menses, or other body fluids. In addition to the washable absorbent assembly 10, the washable absorbent hygienic underwear also comprises a textile pant that has a front region 3, a back region 4, and an intermediate region 7 arranged between the front and back regions 3,4. Further, the washable absorbent assembly 10 is permanently secured in the textile undergarment.

While the invention has been described herein by reference to certain embodiments, it is to be understood that modifications in addition to those described herein may be made to the structures and techniques described herein without departing from the spirit and scope of the invention. Accordingly, although specific embodiments have been described, they are examples only and are not limiting upon the scope of the invention.

## Claims

1. A washable absorbent assembly (10) adapted to be permanently or detachably secured in a textile undergarment (1), the washable absorbent assembly (10) extending a length in a longitudinal direction (y) and a width in a transverse direction (x), having a thickness in a z-direction perpendicular to the longitudinal and transverse directions (x, y), and comprising:
a liquid permeable layer (101),
a liquid barrier member (103) comprising at least one layer, and
an absorbent member (102) arranged between the liquid permeable layer (101) and the liquid barrier member (103),
wherein the liquid barrier member (103) has a breathability level in a range of 2500 to 5500 g/m² /24 hours as measured according to Nonwoven Standard Procedure NWSP 70.4R0 (15).

2. The washable absorbent assembly (10) according to claim 1, wherein the liquid barrier member (103) has a breathability level of 3000 to 5000 g/m² /24 hours.

3. The washable absorbent assembly (10) according to claim 1 or claim 2, wherein the liquid barrier member (103) comprises a plastic film (1032).

4. The washable absorbent assembly (10) according to claim 3, wherein the plastic film (1032) comprises polyurethane.

5. The washable absorbent assembly (10) according to claim 3 or clam 4, wherein the plastic film (1032) is microporous.

6. The washable absorbent assembly (10) according to any one of claims 3 to 5, wherein the plastic film (1032) has a basis weight of 20 to 50 gsm.

7. The washable absorbent assembly (10) according to any preceding claim, wherein the absorbent member (102) comprises a liquid retaining structure (1022) and a liquid acquisition structure (1021) arranged between the liquid permeable layer (101) and the liquid retaining structure (1022), and the liquid retaining structure (1022) has a higher density than the liquid acquisition structure (1021).

8. The washable absorbent assembly (10) according to claim 7, wherein the liquid acquisition structure (1021) has a liquid retaining capacity of 0.2 g/cm² or less and the liquid retaining structure (1022) has a liquid retaining capacity of 0.3 g/cm² or more.

9. The washable absorbent assembly (10) according to claim 7 or claim 8, wherein the liquid retaining structure (1022) and the liquid acquisition structure (1021) each have a respective density, and the respective density of the liquid retaining structure (1022) is 20% higher, preferably 50% higher, than the respective density of the liquid acquisition structure (1021).

10. The washable absorbent assembly (10) according to any preceding claim, wherein the liquid barrier member (103) is a laminate that comprises at least one plastic film (1032) and at least one additional layer (1031).

11. The washable absorbent assembly (10) according to claim 10, wherein the at least one additional layer (1031) is a textile layer.

12. The washable absorbent assembly (10) according to claim 10 to claim 11, wherein the at least one additional layer (1031) is arranged proximal to the absorbent member (102).

13. The washable absorbent assembly (10) according to any preceding claim, wherein the washable absorbent assembly (10) is a reusable absorbent pad adapted to be detachably secured to a textile undergarment.

14. The washable absorbent assembly (10) according to any preceding claim, wherein the washable absorbent assembly (10) does not comprise superabsorbent material.

15. A washable absorbent hygienic underwear for absorbing urine, menses, or other body fluids, the washable absorbent hygienic underwear having a length in a longitudinal direction (y), a width in a transverse direction (x), and a thickness in a z-direction perpendicular to the longitudinal and transverse directions (x, y), and comprising:
a washable absorbent assembly (10) according to any one of claims 1 to 14, and
a textile undergarment comprising a front region (3), a back region (4), and an intermediate region (7) arranged between the front and back regions (3,4),
wherein the washable absorbent assembly (10) is permanently secured in the textile undergarment.
